Europäisches Patentamt

European Patent Office

Office européen des brevets

(11) Publication number: **0 075 128**
**B1**

## (12) EUROPEAN PATENT SPECIFICATION

(45) Date of publication of patent specification: **08.01.86**

(21) Application number: **82107810.2**

(22) Date of filing: **25.08.82**

(51) Int. Cl.⁴: **A 23 L 3/34**, A 23 L 2/30,
A 23 F 5/16, A 24 B 15/28

(54) Method for inactivating mutagens.

(30) Priority: **12.09.81 JP 144272/81**

(43) Date of publication of application:
**30.03.83 Bulletin 83/13**

(45) Publication of the grant of the patent:
**08.01.86 Bulletin 86/02**

(84) Designated Contracting States:
**AT BE CH DE FR GB IT LI LU NL SE**

(56) References cited:
**DE-A-1 517 320**
**DE-A-1 517 333**
**US-A-4 183 364**

(73) Proprietor: **Suntory Limited**
**1-40 Dojimahama 2-chome Kita-ku**
**Osaka-shi Osaka-fu 530 (JP)**

(72) Inventor: **Nagao, Minako**
**Maeda-haitsu 855 No. 511-2, Maeda-cho**
**Totsuka-ku Yokohama-shi Kanagawa (JP)**
Inventor: **Suwa, Yoshihide**
**No. 7-16, Yamatedai 6-chome**
**Ibaraki-shi Osaka (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

# 0 075 128

**Description**

This invention relates to a method for inactivating mutagens contained in foods, foodstuffs, food additives and beverages.

Recently, a survey relating to the causes of cancer in human tissue or organs has caused public attention to be drawn to many mutagens which cause mutation in living organisms. It is still not clear whether every mutagen is a carcinogen. However, it is at least an established fact that there is a close qualitative correlation between *in vitro* mutagenicity and *in vivo* carcinogenicity. Therefore, there have been designed some biological assays for mutagenicity such as "Ames test" in which reverse mutation of microorganisms is used as the parameter [Ames et al., *Mutation Research,* Vol. 31, pp. 347—364 (1975)]. Such methods have broadly been utilized for the preliminary detection of carcinogens in every-day substances.

However, even though such detections or assays are very precise it is not possible to consider factors which effect living things. Moreover, some of the material which may be carcinogens have been used since ancient times. Therefore, it is legally difficult to ban them even though they contain mutagens.

Some examples of foods which are proved to contain carcinogens or mutagens are:

Tobaccos; baked foods such as baked fish, beefsteaks, baked meat and hamburgers. Some drinks which are believed to contain carcinogens or mutagens are: caffeine drinks such as coffee, black tea, etc., alcoholic drinks such as brandy, bourbon and rum.

Even some natural foods *per se*, such as bracken ferm, are believed to possess carcinogenicity (Evans et al., 1965; Hirono et al., 1975). In addition to foods and foodstuffs, it is well-known that tobacco tar contains some carcinogens such as 3,4-benzpyrene.

The presence of mutagens in many foods and foodstuffs is very common. These foods are eaten even though it is undesirable with respect to preventive medicine and eugenics. It would be extremely difficult to halt the consumption of such foods. Particularly, if the food is itself a natural food, it would be nearly impossible to legally ban the consumption of such foods.

The object of the present invention is to increase the safety of foods. Accordingly, another object is to eliminate or inactivate mutagens contained in such foods. Several attempts have previously been made to decrease the mutagen of various materials such as tobacco, for example, by the addition of tannic acid or sugars, as disclosed in Japanese Patent Application (OPI) Nos. 15681/81, 136597/78 and 91197/78 (the term "OPI" as used herein refers to a "published unexamined Japanese patent application"). However, such previous attempts have been ineffective against mutagens in foods and foodstuffs.

As a result of our earnest investigations regarding the inactivation of mutagens in foods, we have found that inorganic sulfites, acidic sulfites-(hydrogen sulfites), pyrosulfites and dithionites or sulfurous anhydride have a strong effect as desmutagens. Among these inorganic compounds, sodium sulfite, sodium hydrogen sulfite, sodium dithionite and sulfurous anhydride or the like have been used for purposes such as bleaching agents and preservatives or antioxidants for foods. Therefore, these materials are available as food additives. However, the use of such materials as desmutagen to suppress mutagenic properties for foods is entirely new.

In accordance with the present invention the mutagenicity of diacetyl or glyoxal can be inactivated by applying at least about 3 molar equivalents of sulfurous acid ion ($SO_3^{--}$) to said substance. The mutagenicity of coffee (irrespective of whether it is regular or instant) can be entirely or nearly inactivated in the presence of sulfites of 20 to 240 ppm as sulfur dioxide. Similarly, the mutagenicity of an alcoholic beverage such as bourbon whisky, apple brandy and rum or the like can also be inactivated entirely or nearly by the addition of 20 to 300 ppm of sulfites.

The present inventors have also found that the above desmutagenic action is almost equally demonstrated with acidic sulfites ($MHSO_3$) ("M" is a monovalent metal atom here and below), pyrosulfites ($M_2S_2O_5$) and dithionites ($M_2S_2O_4$) as well as sulfites and sulfur dioxide (sulfurous anhydride) disclosed above. In addition, it has been found that within the range of the above concentration, the addition of such sulfur-containing inorganic compounds do not affect the flavor and taste of the treated foods.

Therefore, based on the above finding, the present invention is directed to a desmutagen essentially containing sulfites, acidic sulfites, pyrosulfites, dithionites or sulfurous anhydride as the effective agent, as well as the use of such compounds on foods and foodstuffs, food additives and beverages.

In this invention, the salts of sulfur-containing acids except sulfurous anhydride may be used in the form of nontoxic metallic salts. For example, it is possible to use an alkali metal salt such as a sodium or potassium salt; an alkaline earth metal salt such as a calcium or magnesium salt; and an aluminum salt, or an ammonium salt. It should be noted that sodium salts and potassium salts are particularly useful in view of their water-solubility and stability as well as the fact that it is now possible to use such materials as food additives. Typical examples of alkali metal or alkaline-earth metal salts of sulfur-containing acids which can be used in the present invention are sulfites such as $Na_2SO_3$, $K_2SO_3$, $CaSO_3$, $MgSO_3$, $Al(OH)_4SO_3$, $(NH_4)_2SO_3$ and the like, hydrogen sulfites such as $KHSO_3$, $NaHSO_3$, $Ca(HSO_3)_2$ and the like, pyrosulfites such as $K_2S_2O_5$, $Na_2S_2O_5$, $(NH_4)_2S_2O_5$ and the like, dithionites such as $Na_2S_2O_4$, $(NH_4)_2S_2O_4$ and the like. Of these inorganic sulfites, $Na_2SO_3$ and $NaHSO_3$ are preferred. Table 1 summarizes the constitutions and decomposing points of the typical salts available. (The figures in the brackets show the decomposing temperature).

2

TABLE 1

|  | Anion | | | |
|---|---|---|---|---|
| Cation | $SO_3^{--}$ | $HSO_3^{--}$ | $S_2O_5^{--}$ | $S_2O_4^{--}$ |
| $K^+$ | $K_2SO_3$ (decomp.) | $KHSO_3$ (190°C) | $K_2S_2O_5$ (190°C) | — |
| $Na^+$ | $Na_2SO_3$ (decomp.) | $NaHSO_3$ (decomp.) | $Na_2S_2O_5$ (>150°C) | $Na_2S_2O_4$ (unstable) |
| $Ca^{++}$ | $CaSO_3$ (650°C) | $Ca(HSO_3)_2$* | — | — |
| $Mg^{++}$ | $MgSO_3$ (>200°C) | — | — | — |
| $Al^{+++}$ | $Al_2(OH)_4SO_3$ (100°C) | — | — | — |
| $NH_4^+$ | $(NH_4)_2SO_3$ (decomp.) | $NH_4HSO_3$ (decomp.) | $(NH_4)_2S_2O_5$ (150°C) | $(NH_4)_2S_2O_4$ (decomp.) |

* Only present in the form of solution.

The desmutagenic compounds used in the present invention are usually in the form of a powder or solution and are applied to foods by mixing homogeneously therewith. However, fumigation with gaseous $SO_2$ in a closed chamber is also effective, if desired. Alternatively, if the object is coffee, the above desmutagens may be mixed with sugars, coffee cream (coffee whitener) or powdered cream or the like in the form of powder or solution. If the object is regular coffee, the filter paper or cloth which is used for filtering percolate (or eluate) from coffee beans may be impregnated with appropriate salts solutions.

The present invention is further illustrated more in detail by the following Example.

Example
I. Measurement of mutagenicity and desmutagenic effect
(i) Method:

The measurements were carried out using the method according to Sugimura and Nagao, *Chemical Mutagens,* Vol. 6, p. 41 (1981).

(ii) Strain used:

Salmonella typhimurium TA 100 (which requires histidine; hereinafter abbreviated as "S. TA 100). [Ames, McCann and Yamasaki, *Mutation Research,* Vol. 31, pp. 347—364 (1975)].

(iii) Preparation of samples:

(a) In case of chemical substances: Diacetyl or glyoxal is dissolved in aseptic distilled water (the same as below). On the other hand, predetermined amounts of sodium sulfite, sodium hydrogen sulfite (acidic sodium sulfite), pyrosulfite, sodium dithionite and sulfurous anhydride were dissolved in aseptic distilled water, respectively. 50 μl of each of the two solutions was admixed together.

(b) The following examples relates to commercial roasted coffee beans. Commercially-roasted coffee beans were finely pulverized and then extracted with hot distilled water (250 ml per 20 g of milled coffee beans). The extract was then filtered with a commercial coffee filter paper ("Kalita® No. 12", Kalita Co., Ltd., Japan). The filtrate was freeze-dried and then dissolved into distilled water after determining its dry weight. On the other hand, predetermined amounts of sodium sulfite, sodium hydrogen sulfite, potassium pyrosulfite, sodium dithionite and sulfurous anhydride were dissolved in distilled water, respectively. 50 μl of each of the two solutions were then admixed together.

(c) the following example relates to self-roasted coffee beans.

(c-1) Roasting coffee beans
Kind of beans: *Coffea arabica*, Santoz No. 2
Roaster: "Lucky Coffee Roaster" (direct gas-heating type, capacity 3.6 kg, SLR No. 2)
Roasting conditions: 3.6 kg of beans was used per each roasting.

3

| Degree of the roasting | Roasting time | Weight of the beans roasted |
|---|---|---|
| A: Light | 10 Minutes | 2.6 kg |
| B: Medium | 14 Minutes | 2.5 kg |
| C: Heavy | 18 Minutes | 2.3 kg |

(c-2) Preparation of coffee extract

To 500 g of fine roasted coffee powder, there was added 4 l of hot water. This was boiled for 10 minutes and then filtered with cloths. To the filtrate, an equal quantity of fine coffee powder was newly added and this was boiled for an additional 10 minutes. To this boiled extract, 500 g of new roasted coffee powder was added. This was boiled for another 10 minutes and then filtered to obtain the testing coffee extract.

(c-3) Addition of sulfurous acid

The solid matters in the above coffee extract were measured by an "ATAGO DEGITAL REFRACTOMETER MODEL DBX-50". Three levels of anhydrous sodium sulfite (food additive grade, 98% purity) were added to the solid matter to form a solution.

(c-4) Pulverization

(c-4-1) Spray-dry method: The above sulfite-added coffee extract was spray-dried with a minipilot spray dryer ("Yamato MINISPRAY Model 60R") under the following conditions in order to give 25 g of a dry sample from 500 g of starting extract. Inlet temperature: 110 to 130°C, Outlet temperature: 52 to 57°C, Dry air: 0.45 $m^3$/min, Neblized air: 11 l/min. The sample extract was provided at a rate of 88 ml/min.

(c-4-2) Freeze-dry method: The above sulfite-added coffee extract was freeze-dried over a minifreeze-dryer (MS Freeze-Dryer Model 60R) under the following conditions in order to obtain 10 g of a dry sample from 100 g of extract.

Coolant: methanol

Coolant temperature: −50 to 55°C

Vacancy: $6,6 \times 10^{-4}$ mbar

(d) An example using instant coffee is now described. The tested instant coffee was dissolved in distilled water. On the other hand, predetermined amounts of potassium sulfite, sodium hydrogen sulfite, potassium pyrosulfite, sodium dithionite or sulfurous anhydride were dissolved in water, respectively, and 50 µl of each of the solutions were mixed.

(e) An example using an alcoholic beverages is now described. Predetermined amounts of sodium sulfite, sodium hydrogen sulfite, potassium pyrosulfite, sodium dithionite and sulfurous anhydride were dissolved in 1.0 ml of distilled water, respectively. The solutions thus obtained were immediately added to alcoholic beverages, and the mixtures were allowed to stand for 30 minutes. Samples of these beverages having a volume of 25 to 50 ml were dried over a rotary evaporator at less than 40°C and the dry matter from each sample was then dissolved into 1 ml of DMSO.

(iv) Measurement of the mutagenicity

To 100 µl of each sample obtained by subparagraphs (a) to (f), (iii), aforementioned 0.1 M sodium phosphate buffer (pH 7.4) and 0.1 ml of the Salmonella culture (aforementioned TA 100 strain) were added. The mixture was incubated for 20 minutes at 37°C and then added onto 2 ml of soft agar. Thereafter the second mixture was spread on 0.1% glucose agar plate. To the above soft agar was added previously histidine in an amount of 0.1 µmole/2 ml of soft agar/plate which is necessary for several fissions of the bacteria on the plate. The plate was allowed to incubate for 48 hours at 37°C and then the number of colonies per $10^8$ surviving bacteria cells was enumerated as the mutant. The inactivation rate was calculated by the following formula:

$$\text{Inactivation Rate} = \left(1 - \frac{\text{number of colonies on the plate with desmutagen}}{\text{number of colonies on the plate without any desmutagen}}\right) \times 100 \ (\%)$$

II. Results

(i) Inactivation of 1,2-dicarbonyls by sodium sulfite

The 1,2-dicarbonyl compound diacetyl is reported to be present in coffee (Gianturco et al., 1966). Since diacetyl is volatile, it may not be the main mutagen in coffee. (See, method for preparation of freshly-brewed coffee powder.) However, diacetyl and its related compound glyoxal are direct-acting

## 0 075 128

mutagens to S. TA 100 like coffee. They were mixed with an excess molar concentration of sulfite. The results obtained are shown in Tables 2 and 3. From the tables, it is clear that both the mutagenicities of glyoxal and diacetyl are completely inactivated by addition of 8.0 mM and 12.0 mM, respectively, of sodium sulfite against 2.81 mM of the former and 4.20 mM of the latter, respectively. Therefore, the addition of sodium sulfite would be recommendable to inactivate known mutagens diacetyl and glyoxal in foods and drinks.

### TABLE 2

| Glyoxal (mM) | Sodium sulfite (mM) | Number of revertants/ $10^8$ survivors* |
|---|---|---|
| 1.12 | 0 | 126 |
| 2.81 | 0 | 335 |
| 5.61 | 0 | 661 |
| 1.12 | 8.0 | 0 |
| 2.81 | 8.0 | 0 |
| 5.61 | 8.0 | 41 |

* The number of spontaneous revertant (150) was subtracted (hereinafter the same).

### TABLE 3

| Diacetyl (mM) | Sodium sulfite (mM) | Number of revertants/ $10^8$ survivors* |
|---|---|---|
| 1.65 | 0 | 220 |
| 4.20 | 0 | 398 |
| 1.65 | 12 | 0 |
| 4.20 | 12 | 0 |

* The same as Table 2.

(ii) Inactivation of the mutagenic activities of regular coffee by the salts of sulfur-containing acid (Part 1)

Each 2 mg, 5 mg, 10 mg, 15 mg and 20 mg of powdered coffee beans extract prepared by the foregoing method (iii), (b) were dissolved in distilled water. Each of the dissolved extracts made 50 μl of solution. On the other hand, the salts of sulfur-containing acids were dissolved in distilled water in order to make 50 μl containing 240 μg of sulfur dioxide.

The solutions were admixed together and the mixture was further admixed with 0.5 ml of 0.1 M sodium phosphate buffer (pH 7.4) and the above-described 0.1 ml of the culture of S. TA 100. The mixture was then incubated in the same manner as described in (I) above. The results obtained are shown in Table 4.

### TABLE 4

| Coffee (mg/plate) | Salts of sulfur-containing acid | | | | |
|---|---|---|---|---|---|
| | None (control) | Sodium sulfite | Sodium hydrogen sulfite | Potassium pyrosulfite | Sodium dithionite |
| 2 | 53 | 0 | 0 | 0 | 0 |
| 5 | 102 | 0 | 0 | 0 | 0 |
| 10 | 212 | 0 | 0 | 0 | 0 |
| 15 | 363 | 10 | 0 | 0 | 0 |
| 20 | 601 | 10 | 0 | 0 | 0 |

5

# 0 075 128

As shown in Table 4, regular (freshly-brewed) coffee lost mutagenicity on S. TA 100 without S9 mix when sodium sulfite was added. S9 mix is required for the metabolic activation of promutagen [*Pro. Natl. Acad. Sci. USA,* Vol. 70 (8), pp. 2281—2285 (1973)]. Killing effect of coffee on S. TA 100 was also suppressed by addition of sodium sulfite. Brewed (regular) or instant coffee made in the ordinary way contains 5—15 mg of dry matter per ml. Therefore, from Table 4, it can be concluded that the mutagenicities of coffee served on the table were inactivated by adding sulfite at 300 ppm.

Coffee was weakly mutagenic on *S. typhimurium* TA98 without S9 mix (about 9 revertants/mg of instant coffee) and this mutagenic activity was also inactivated by sodium sulfite at the same concentration.

Addition of sodium sulfite did not result in formation of any mutagen that was detectable with S. TA 100 or TA 98 and S9 mix.

We also tested the effects of sodium hydrogen sulfite, potassium pyrosulfite and sodium dithionite, since these compounds are also used as food additives. These compounds were added in the same molar concentration (3.75 mM) as sulfite (300 ppm) and they also inactivated the mutagenicity of regular coffee. Since pyrosulfite ion was rapidly converted to hydrogen sulfite ion in water, these chemicals seemed to react with the mutagenic principle(s) in coffee as hydrogen sulfite.

(iii) Inactivation of the mutagenic activities of regular coffee by sodium sulfite (Part 2)

The result of the mutagenicity test in sodium sulfite-added coffee powder (spray-dried and freeze-dried) prepared by the above-described method (iii), (c) was set forth in the following Table 5.

## TABLE 5

| Degree of roasting | Drying[1] method | Amount of[2] $SO_3$ added | Number of revertants[3] $10^8$ survivors | Number of revertants[4] per one-cup of instant coffee/$10^8$ survivors |
|---|---|---|---|---|
| Light | S | 0 | 334 | 66,800 |
| " | F | 0 | 464 | 92,800 |
| " | S | 4 | 124 | 24,800 |
| " | F | 4 | 117 | 23,400 |
| " | S | 20 | 0 | 0 |
| " | F | 20 | 0 | 0 |
| Medium | S | 0 | 1,551 | 310,200 |
| " | F | 0 | 1,208 | 241,600 |
| " | S | 4 | 643 | 128,600 |
| " | F | 4 | 626 | 125,200 |
| " | S | 20 | 0 | 0 |
| " | F | 20 | 0 | 0 |
| Heavy | S | 0 | 586 | 117,200 |
| " | F | 0 | 454 | 90,800 |
| " | S | 4 | 565 | 113,000 |
| " | F | 4 | 552 | 110,400 |
| " | S | 20 | 0 | 0 |
| " | F | 20 | 0 | 0 |

[1] S: Spray-dried; F: Freeze-dried
[2] $SO_3$ mg per gram of solid matter
[3] per 15 mg of powdered coffee
[4] per one cup of instant coffee [as 150 ml/cup (solid matter 3 g)]

6

As shown in Table 5, even in the case of medium-roasted coffee which is highest in mutagenicity, the mutagenicity is inactivated by the addition of 20 mg of $SO_3$ per 1 g of powdered coffee extract.

(iv) The desmutagenic effect of the salts of sulfur-containing acids on instant coffee (Part 1) is described below.

Each 2.5 mg, 5 mg, 10 mg and 15 mg of commercially-powdered instant coffee was dissolved in distilled water to make 50 µl of the solution, respectively. On the other hand, a predetermined amount of sodium sulfite was dissolved into distilled water in order to make 50 µl of the solution in which the sulfite was contained at concentrations of 0 ppm, 25 ppm, 50 ppm, 80 ppm and 240 ppm as sulfur dioxide, respectively.

The two solutions were mixed together and to the mixture there was added 0.5 ml of phosphate buffer solution and 0.1 ml of the culture as in the regular coffee. The above-described test was then repeated five times. The results are shown in the following Table 6. The numerations in the table are the average values of five tests for each.

TABLE 6

| Added $SO_2$ (ppm) | 0 | 25 | 50 | 80 | 240 |
|---|---|---|---|---|---|
| Remaining $SO_2$ (ppm) | 0 | 10 | 15.9 | 41.6 | 190 |
| Amount of Coffee (mg/plate) | | | | | |
| 2.5 | 24 | 18 | 7* (71) | 3* (87) | 0* (100) |
| 5.0 | 77 | 39 | 10* (87) | 3* (96) | 0* (100) |
| 12.0 | 223 | 140 | 70* (69) | 59* (74) | 0* (100) |
| 15.0 | 290 | 274 | 230 (21)* | 162* (44) | 0* (100) |

* Figures in brackets show the inhibition rate.

The brewed or instant coffee made in ordinary way generally contains 5 to 15 mg of dry matter per ml. Therefore, from Table 6, it can be concluded that the mutagenicities of coffee served on the table were completely inactivated by addition of sulfite at a concentration of 240 ppm.

(v) The desmutagenic effect of the salts of sulfur-containing acids on instant coffee (Part 2)

The following Table 7 shows the results obtained by another test in which sodium sulfite, equivalent sodium hydrogen sulfite, potassium pyrosulfite, sodium dithionite and sulfurous anhydride were added to an instant coffee to make a 240 ppm concentration of sulfur dioxide.

As seen from the table, by adding this amount, the mutagenicity against S. TA 100 is completely eliminated. A blind test was performed to show that the addition of the sulfite at a concentration of 240 ppm as sulfur dioxide equivalent had no effects on the growth of the above S. TA 100 strain.

TABLE 7

| Amount of coffee powder (mg) | Not added (control) | Salts of sulfur-containing acids | | | | |
|---|---|---|---|---|---|---|
| | | Sodium sulfite | Sodium hydrogen sulfite | Potassium pyrosulfite | Sodium dithionite | Sulfurous anhydride |
| 2.5 | 39 | 0 | 0 | 0 | 0 | 0 |
| 5 | 92 | 0 | 0 | 0 | 0 | 0 |
| 10 | 241 | 0 | 0 | 0 | 0 | 0 |
| 15 | 310 | 0 | 0 | 0 | 0 | 0 |
| 20 | 403 | 0 | 0 | 0 | 0 | 0 |

From the results shown in Table 7, it can be concluded that the mutagenicities of instant coffee were completely inactivated by addition of the salts of sulfur-containing acids at a concentration of 240 ppm as sulfur dioxide equivalent.

(vi) Inactivation of the mutagenic properties in alcoholic beverages by sulfurous anhydride (Part 1)

To commercial bourbon whisky, there were added sulfurous anhydride in order to make concentrations of 20 ppm, 60 ppm, 125 ppm and 250 ppm of sulfur dioxide equivalent, respectively. The results are shown in the following Table 8. As seen from the table, by addition of 250 ppm of sulfurous anhydride (as sulfur dioxide), the mutagenicity of the samples is completely eliminated.

TABLE 8

| Amount of $SO_3$ Added (ppm) | 0 | 20 | 60 | 125 | 250 |
|---|---|---|---|---|---|
| Amount of Bourbon (ml equivalent/plate) | | | | | |
| 0.25 | 22 | 20 | 21 | 19 | 0 |
| 0.625 | 281 | 220 | 180 | 40 | 0 |
| 1.25 | 800 | 740 | 440 | 140 | 0 |
| 1.875 | 1,060 | 1,040 | 680 | 240 | 0 |
| 2.50 | 1,660 | 1,440 | 860 | 380 | 0 |

(vii) Inactivation of the mutagenicity in alcoholic beverages by sulfites and sulfurous anhydride (Part 2)

To commercial calvados (apple brandy), sodium sulfite and sulfurous anhydride were added corresponding to the amount of 150 ppm as sulfur dioxide. The results are shown in the following Table 9. As understood from the Table, the mutagenicity of calvados are completely inactivated by addition of sodium sulfite or sulfurous anhydride corresponding to 150 ppm as sulfur dioxide.

TABLE 9

| Calvados (ml equivalent/ plate) (ppm as $SO_2$) | Sodium sulfite (ppm as $SO_2$) | Number of revertants/ plate | Sulfurous anhydride (ppm as $SO_2$) | Number of revertants/ plate |
|---|---|---|---|---|
| 0.5 | 0 | 20 | | |
| 1.25 | 0 | 88 | | |
| 2.5 | 0 | 104 | | |
| 3.75 | 0 | 364 | | |
| 0.5 | 150 | 0 | 150 | 0 |
| 1.25 | 150 | 0 | 150 | 0 |
| 2.5 | 150 | 0 | 150 | 0 |
| 3.75 | 150 | 0 | 150 | 0 |

(viii) Inactivation of the mutagenicity in alcoholic beverages by sulfites (Part 3)

To commercial rum, sodium sulfite was added corresponding to 150 ppm in order to survey the desmutagenic effect according to the above-described method I. (iv). The results are shown in the following Table 10. As seen from the table, the mutagenicity of 2.5 ml equivalent per plate of samples is completely eliminated by addition of sodium sulfite corresponding to the amount of 150 ppm of sulfurous anhydride.

**0 075 128**

TABLE 10

| Rum (ml equivalent/ plate) | Sodium sulfite (ppm as $SO_2$) | Number of revertants/plate |
|---|---|---|
| 0.25 | 0 | 11 |
| 0.625 | 0 | 55 |
| 1.25 | 0 | 98 |
| 1.875 | 0 | 263 |
| 2.5 | 0 | 502 |
| 0.25 | 150 | 0 |
| 0.625 | 150 | 0 |
| 1.25 | 150 | 0 |
| 1.875 | 150 | 0 |
| 2.5 | 150 | 0 |

There are many epidemiological studies which demonstrate a correlation between alcohol consumption and cancer of the upper alimentary tract or the upper respiratory tract. Especially, it has been reported that the incidence of esophageal cancer is high in such places as France, Puerto Rico and U.S.A. where calvados, rum and bourbon whisky are consumed respectively in relatively high quantities, and therefore such alcoholic drinks are considered to contain cancer-inducing materials. In fact, these spirituous liquors contain mutagenicities in an amount relatively higher than that found in other spirituous liquors. Thus, the inactivation of mutagenicity in these liquors would be advantageous from the standpoint of decrease in incidence of cancer.

Another advantage of the present invention is that the addition of desmutagen according to the present invention at a level effective as desmutagen has no adverse effect on the taste and the flavor of foods and drinks.

As described hereinbefore in detail, since the present invention is distinctly effective in order to inactivate mutagens in various foods and beverages.

Accordingly, the present invention can greatly improve human health, eugenics and preventive medicine by increasing the safety of foods.

**Claims**

1. A method for inactivating mutagens contained in foods, foodstuffs, food additives and beverages which comprises contacting sulfurous anhydride ($SO_2$) or an alkali metal, alkaline earth metal, aluminum or ammonium salt of sulfite, hydrogen sulfite, pyrosulfite or dithionite with foods, foodstuffs, food additives and beverages at a concentration sufficient to inactivate mutagens contained in said foods, foodstuffs, food additives and beverages.

2. The method according to Claim 1, wherein said salt of sulfite is $K_2SO_3$, $Na_2SO_3$, $CaSO_3$, $MgSO_3$, $Al_2(OH)_4SO_3$ or $(NH_4)_2SO_3$.

3. The method according to Claim 1, wherein said salt of hydrogen sulfite is $KHSO_3$, $NaHSO_3$ or $Ca(HSO_3)_2$.

4. The method according to Claim 1, wherein said salt of pyrosulfite is $K_2S_2O_5$, $Na_2S_2O_5$ or $(NH_4)_2S_2O_5$.

5. The method according to Claim 1, wherein said salt of dithionite is $Na_2S_2O_4$ or $(NH_4)_2S_2O_4$.

6. The method according to Claim 1, wherein said salt of sulfite is $Na_2SO_3$.

7. The method according to Claim 1, wherein said salt of hydrogen sulfite is $NaHSO_3$.

**Patentansprüche**

1. Verfahren zur Inaktivierung von in Lebensmitteln, Nahrungsstoffen, Lebensmittelzusatzstoffen und Getränken enthaltenen Mutagenen, dadurch gekennzeichnet, dass man Schwefligsäureanhydrid ($SO_2$) oder ein Alkalisalz, Erdalkalisalz, Aluminium- oder Ammoniumsalz von Sulfit, Hydrogensulfit, Pyrosulfit oder Dithionit mit Lebensmitteln, Nahrungsstoffen, Lebensmittelzusatzstoffen und Getränken in einer

9

ausreichenden Konzentration in Kontakt bringt, um die in den genannten Lebensmitteln, Nahrungsstoffen, Lebensmittelzusatzstoffen und Getränken enthaltenen Mutagene zu inaktivieren.

2. Verfahren nach Anspruch 1, dadurch gekennzeichnet, dass das genannte Sulfitsalz $H_2SO_3$, $Na_2SO_3$, $CaSO_3$, $MgSO_3$, $Al_2(OH)_4SO_3$ oder $(NH_4)_2SO_3$ darstellt.

3. Verfahren nach Anspruch 1, dadurch gekennzeichnet, dass das genannte Hydrogensulfit $KHSO_3$, $NaHSO_3$ oder $Ca(HSO_3)_2$ darstellt.

4. Verfahren nach Anspruch 1, dadurch gekennzeichnet, dass das genannte Pyrosulfitsalz $K_2S_2O_5$, $Na_2S_2O_5$ oder $(NH_4)_2S_2O_5$ darstellt.

5. Verfahren nach Anspruch 1, dadurch gekennzeichnet, dass das genannte Dithionitsalz $Na_2S_2O_4$ oder $(NH_4)_2S_2O_4$ darstellt.

6. Verfahren nach Anspruch 1, dadurch gekennzeichnet, dass das genannte Sulfitsalz $Na_2SO_3$ darstellt.

7. Verfahren nach Anspruch 1, dadurch gekennzeichnet, dass das genannte Hydrogensulfitsalz $NaHSO_3$ darstellt.

**Revendications**

1. Un procédé pour l'inactivation de mutagènes contenus dans des aliments, des comestibles, des additifs pour produits alimentaires et des boissons, qui consiste à mettre en contact de l'anhydride sulfureux ($SO_2$) ou un sulfite, un hydrogénosulfite, un pyrosulfite ou un dithionite de métal alcalin, de métal alcalino-terreux, d'aluminium ou d'ammonium avec des aliments, des comestibles, des additifs pour produits alimentaires et des boissons à une concentration suffisante pour inactiver les mutagènes contenus dans lesdits aliments, comestibles, additifs pour produits alimentaires et boissons.

2. Procédé selon la revendication 1, dans lequel ledit sulfite est le $K_2SO_3$, le $Na_2SO_3$, le $CaSO_3$, le $MgSO_3$, l'$Al_2(OH)_4SO_3$ ou le $(NH_4)_2SO_3$.

3. Procédé selon la revendication 1, dans lequel ledit hydrogénosulfite est le $KHSO_3$, le $NaHSO_3$ ou le $Ca(HSO_3)_2$.

4. Procédé selon la revendication 1, dans lequel ledit pyrosulfite est le $K_2S_2O_5$, le $Na_2S_2O_5$ ou le $(NH_4)_2S_2O_5$.

5. Procédé selon la revendication 1, dans lequel ledit dithionite est le $Na_2S_2O_4$ ou le $(NH_4)_2S_2O_4$.

6. Procédé selon la revendication 1, dans lequel ledit sulfite est le $Na_2SO_3$.

7. Procédé selon la revendication 1, dans lequel ledit hydrogénosulfite est le $NaHSO_3$.